# EUROPEAN PATENT SPECIFICATION

(11) **EP 2 018 839 B1**
(45) Date of publication and mention of the grant of the patent: **28.12.2011**
(21) Application number: 08159519.1
(22) Date of filing: 02.07.2008
(51) Int. Cl.: A61K 8/19, A61K 8/34, A61K 8/362, A61Q 17/00

(54) **Preservative cosmetic composition comprising electrolytic colloidal silver**
Kosmetisches Konnservierungsmittel enthaltend ein elektrolytisches kolloidales Silber
Composition conservatrice cosmétique comprenant de l'argent électrolytique colloïdal

(30) Priority: 17.07.2007 IT PD20070242
(43) Date of publication of application: 28.01.2009
(73) Proprietor: Farmogal S.R.L., 35030 Rovolon Bastia (PD) (IT)
(72) Inventor: Zago, Cristian, 30030, Cazzago Di Painiga VE (IT); Galiano, Fabio, 36020, Albettone VI (IT)
(74) Representative: Modiano, Micaela Nadia

(56) References cited:
- US-A1- 2007 053 850
- HASSELT VAN P ET AL: "COLLOIDAL SILVER AS AN ANTIMICROBIAL AGENT: FACT OR FICTION?" JOURNAL OF WOUND CARE, MACMILLAN, LONDON, GB, vol. 13, no. 4, 1 April 2004 (2004-04-01), page 154/155, XP009039635 ISSN: 0969-0700

## Description

The present invention relates to a preservative composition for cosmetic products comprising electrolytic silver.

As is known, many cosmetics include water in their formulation and therefore, in order to inhibit its consequent contamination by microorganisms, cosmetics receive the addition of so-called preservatives.

Although the use of preservatives has solved the problem of bacterial contamination, in many cases, however, it has created other problems, since preservatives have allergic potentials and can cause collateral reactions which include skin irritation, eye irritation, and contact sensitization.

The use of a preservative composition in cosmetic products as known from Italian patent No. 1307312 obviates the problems mentioned above. This composition is represented by a combination of a polyalcohol (glycerol), a pH buffer and a chelating agent, characterized by a pH of about 4. Despite preservation effectiveness and reduced side-effects, it is noted that the acidification of the pH can cause, in predisposed subjects or subjects with a sensitive skin, some form of intolerance to the product, with consequent interruption of the cosmetic treatment. Further, the composition has a limited antibacterial effectiveness against some species of microorganisms, such as Aspergillus niger or Candida albicans, and is not indicated for combination with all types of cosmetics, especially those in which the content of polyalcohols such as glycerol or of chelating agents must be kept within narrower specific limits.

An effective antibacterial agent which is known historically for its positive effects against microorganisms is metallic silver. The antimicrobial action of metallic silver is due to its capacity to penetrate bacterial and fungal membranes of unicellular organisms, binding then to proteins and respiratory enzymes and to nucleic acids, blocking the survival systems of the microorganisms (Berger et al., Antimicrob Agents Chemother., 9(2):357-8, 1976).

Forms of silver such as colloidal silver, silver sulfadiazine, silver arsphenamine, silver nitrate or other salts have been used for therapeutic purposes, but the addition of silver in cosmetic products is usually prohibited due to alteration of the stability and organoleptic characteristics of the product. At the same time, the use of small amounts of silver is instead ineffective in exhibiting a preservative effect.

Further, although silver does not have a toxic action at the level of mammalian cells, the prolonged taking of this metal by superior organisms has some unwanted effects, due to which several countries, including the United States and Australia, have prohibited the therapeutic use and sale of products containing silver. The most evident of these effects is argyria, a condition in which the skin of the affected person assumes irreversibly an unsightly albeit harmless blue-gray coloring (Wadhera A. and Fung M., J Dermatol Online J., 11(1):12, 2005).

There is, therefore, the need to provide a preservative composition which has no allergic or irritating effects and is characterized by a superior antimicrobial effect and can be included in a wide range of cosmetic products in their various forms.

The aim of the present invention is to provide a new preservative composition for cosmetic products which keeps unchanged the organoleptic characteristics and the stability of the cosmetic product despite higher effectiveness against micro-organic flora.

Within this aim, an object of the invention is to provide a composition with a preservative action which can be formulated without preservatives and at a pH which is more compatible with skin physiology.

Another object of the invention is to provide a preservative composition for cosmetics which can be used in combination with cosmetic products in which it is necessary to limit the concentration of polyalcohols such as glycerin.

A further object of the present invention is to provide a preservative composition for cosmetics in which it is necessary to limit, or there is a prohibition against, the use of ethylenediaminetetraacetic acid (EDTA).

This aim and these and other objects which will become better apparent hereinafter are achieved by a preservative composition for cosmetic products which comprises:
5 to 15% by weight of glycerin with respect to the weight of the cosmetic product,
0.01 to 0.5% by weight of chelating compound with respect to the weight of the cosmetic product,
a dynamic buffer for keeping the pH around 4.50-6.00, and
is characterized in that it further comprises
0.1 to 1 ppm of electrolytic colloidal silver with respect to the cosmetic product.

The aim and objects of the invention are also achieved by a cosmetic product which comprises the preservative composition described here.

According to the invention, the preservative composition comprises a quantity of 0.1 to 1 ppm of electrolytic colloidal silver with respect to the cosmetic product. Preferably, the quantity of electrolytic colloidal silver can be 0.5 ppm with respect to the cosmetic product.

Electrolytic colloidal silver is the only form of silver approved as a supplement by the Food and Drug Administration. In the composition according to the invention, the integration of electrolytic colloidal silver with the other components of the preservative composition allows to use a reasonably low quantity of silver, such as to not induce side effects such as for example argyria. Further, since electrolytic colloidal silver is the most powerful known oligodynamic metal with antibacterial properties, antimicrobial effectiveness is ensured even at extremely low concentrations. Advantageously, the reduced quantity of silver does not alter the organoleptic characteristics of the cosmetic product to which it is added.

The formulation of the preservative composition according to the invention provides for the increase of the pH to values which are closer to the physiological value of the skin, which is approximately 5.5. As a consequence of this variation, a substantially complete reduction of forms of intolerance to the cosmetic product caused by the final acidity of the product is observed even in predisposed subjects or subjects with a sensitive skin.

Further, the addition of electrolytic colloidal silver allows to formulate the preservative composition in such a manner as to allow its use also in cosmetic products in which the quantities of polyalcohols such as glycerin or of chelating agents can have unfavorable effects as regards stability.

The preservative composition according to the invention in fact comprises a quantity of glycerin comprised between 5 and 15% by weight with respect to the weight of the cosmetic product, a quantity which, in addition to being lower than the value generally believed to cause irritation (comprised between 30% and 35%), makes the preservative composition with silver suitable for application in cosmetic formulations in which it is fundamental to keep low the contents of polyalcohols such as glycerin in order to improve the pleasantness of the product. Preferably, the quantity of glycerin can be 5% to 10% by weight of the cosmetic product.

The preservative composition according to the invention comprises a chelating agent in a quantity between 0.01 and 0.5% by weight on the weight of the cosmetic product, whose task is to chelate metals such as calcium, magnesium and heavy metals. Preferably, the amount of chelating agent can be 0.05% to 0.3% by weight on the weight of the cosmetic product.

In an embodiment according to the invention, the chelating agent can be ethylenediaminetetraacetic acid (EDTA) and/or salts thereof.

The addition of electrolytic colloidal silver allows to formulate the preservative composition with a reduced quantity of EDTA (0.01-0.5%), allowing to use the composition according to the invention also in cosmetic products in which the quantity of EDTA is prohibited or limited. For example, cosmetics of this type can be cosmetics which contain an additive of the group constituted by an acrylic polymer gel (carbomer gel) and an emulsifier. In compositions without silver with a large quantity of EDTA, the chelating agent acts in fact as a fluidifying agent on the acrylic polymers, preventing them from forming a gel, and interferes with the action of the emulsifiers.

Of course, the EDTA can be replaced with any other chelating agent.

The composition according to the invention further comprises a dynamic buffer for keeping the pH around 4.50-6.00. For example, the buffer can be a citric-phosphate buffer. Preferably, the pH is kept around 5.00-5.50.

This dynamic buffer, which is per se known for example from Italian patent No. 1291417, is a so-called "smart" system in dynamic equilibrium, which ensures that the pH is kept at the preset values, contrasting the changes that microorganisms would be capable of producing on the composition to their own advantage for their growth.

The invention further consists of a cosmetic product which comprises the preservative composition according to the invention. In an exemplifying but non-limiting embodiment, the cosmetic product comprises an active ingredient selected from the group constituted by collagen, royal jelly and hyaluronic acid.

Of course, the cosmetic product can comprise, as an active ingredient, any compound normally used in the cosmetic industry.

In another embodiment, the cosmetic product according to the invention further comprises an additive selected from the group constituted by an acrylic polymer gel and an emulsifier.

Of course, the cosmetic product according to the invention can assume various cosmetic forms and can be for example in the form of a cream, a gel, a solution, a milk or a suspension.

In practice it has been found that the preservative composition according to the invention fully achieves the intended aim and objects, since the addition of electrolytic colloidal silver combined with a pH at physiological level leads to a product which is well tolerated by the skin even in subjects who are predisposed or have a sensitive skin.

Moreover, it has been found that the preservative composition according to the invention is characterized by a superior bactericidal effect, since the addition of electrolytic colloidal silver is capable of reducing the bacterial load below the levels of acceptability according to the pharmacopoeia.

Further, by way of the higher effectiveness against microorganisms, the preservative composition according to the invention ensures, in the cosmetic products to which it is added, an extension of the period after opening (PAO).

It has been also observed that the preservative composition according to the invention can be incorporated within cosmetic products which require a low content of polyalcohols (glycerin) to achieve greater pleasantness, since the combination of the preservative components with the electrolytic colloidal silver allows to keep the quantity of glycerin at a maximum concentration of 10% by weight of the finished product.

Finally, the preservative composition according to the invention, thanks to the incorporation of the electrolytic colloidal silver, allows to keep the concentration of EDTA at lower levels than that in cosmetic products preserved without silver, and is thus effective also in combination with cosmetic products which contain additives such as acrylic polymer gels or emulsifiers on which EDTA has a negative effect.

Further characteristics and advantages of the invention will become better apparent from the following detailed description of an embodiment thereof, given by way of non-limiting example.

### Example

Microbiological analyses were conducted by means of a so-called "challenge test", by using the microorganisms indicated in Tables 1-5, in order to assess the microbiological resistance of three samples represented by a corresponding number of aqueous solutions in which the preservative composition with electrolytic colloidal silver ("Silver" colloidal silver, Fluka - Switzerland) with a physiological pH of 5.5 was added to a cosmetic containing an active ingredient among collagen, royal jelly and hyaluronic acid. A comparative analysis was also performed with three solutions, in which a preservative solution without silver at pH 4.0 was added to the same cosmetics.

The composition of the solutions with electrolytic colloidal silver used is the following:

| | |
|---|---|
| Glycerin | 5.00% by weight of the composition |
| EDTA | 0.50% by weight of the composition |
| Buffer | citric acid 0.20% and dibasic potassium to pH 5.00 |
| Electrolytic silver | 0.50 ppm |
| Cosmetic | q.s. To 100% by weight of the composition. |

As prescribed by the CTFA method, the inoculi are on the order of 10⁷ UFC/ml for bacteria and 10⁵ UFC/ml for yeasts and molds. The inoculi are derived from mother suspensions of microorganism for scalar dilutions such as to obtain dishes with readable inoculi comprised between 30 and 300 UFC/ml.

5 ml of base inoculum were introduced in 45 ml of each solution or emulsion, and the resulting samples were left at ambient temperature and in the dark for the entire duration of the experiment. According to the CTFA method, after 1, 7, 14 and 28 days (T1, T7, T14 and T28) after the initial attack (TO), the counts were performed. For this purpose, scalar dilutions of the samples (1:10) with a phosphate buffer at pH 7.2 were prepared, seeding for each dilution in two plates with PCA media for S. aureus and E. coli, with TSA media for P. aeruginosa and Sabouraud for yeasts and molds in general.

The incubation conditions for microbial growth are, for PCA and TSA, 48 hours in a thermostat at 37°C and five days at ambient temperature for Sabouraud.

The results were read by multiplying by the corresponding dilution the number of colonies counted on each plate, calculating the average of the resulting UFC/ml values.

In the tables, the columns labeled "Comp" refer to comparison solutions with a preservative composition at pH 4.0, the columns "Ag" refer to solutions with the preservative composition with silver at pH 5.5.

**Table 1**

| Aspergillus niger | | | | | | |
|---|---|---|---|---|---|---|
| | Collagen | | Royal jelly | | Hyaluronic acid | |
| | Comp | Ag | Comp | Ag | Comp | Ag |
| T0 | 45000 | 45000 | 45000 | 45000 | 45000 | 45000 |
| T1 | 40000 | 30000 | 5000 | 3000 | 4000 | 400 |
| T7 | 10000 | 6000 | 4000 | 2000 | 2000 | <10 |
| T14 | 3000 | 300 | 2000 | 1000 | 1000 | <10 |
| T28 | 400 | 100 | 500 | <10 | 50 | <10 |

**Table 2**

| Candida albicans | | | | | | |
|---|---|---|---|---|---|---|
| | Collagen | | Royal jelly | | Hyaluronic acid | |
| | Comp | Ag | Comp | Ag | Comp | Ag |
| T0 | 38000 | 38000 | 38000 | 38000 | 38000 | 38000 |
| T1 | 30000 | 36000 | 20000 | 14000 | 25000 | 330 |
| T7 | 4700 | 420 | 650 | 360 | 310 | <10 |
| T14 | 250 | 80 | 10 | <10 | 70 | <10 |
| T28 | <10 | <10 | <10 | <10 | <10 | <10 |

**Table 3**

| Escherichia coli | | | | | | |
|---|---|---|---|---|---|---|
| | Collagen | | Royal jelly | | Hyaluronic acid | |
| | Comp | Ag | Comp | Ag | Comp | Ag |
| T0 | 18700000 | 18700000 | 18700000 | 18700000 | 18700000 | 18700000 |
| T1 | 12300000 | 40000 | 290000 | 4000 | 14000000 | 100 |
| T7 | 2400000 | <10 | <10 | <10 | 6200000 | <10 |
| T14 | 9800 | <10 | <10 | <10 | 14800 | <10 |
| T28 | 50 | <10 | <10 | <10 | <10 | <10 |

**Table 4**

| Staphilococcus aureus | | | | | | |
|---|---|---|---|---|---|---|
| | Collagen | | Royal jelly | | Hyaluronic acid | |
| | Comp | Ag | Comp | Ag | Comp | Ag |
| T0 | 22500000 | 22500000 | 22500000 | 22500000 | 22500000 | 22500000 |
| T1 | 790000 | 610000 | 2000 | <10 | 390000 | <10 |
| T7 | 2200 | 600 | 10 | <10 | 1100 | <10 |
| T14 | 20 | <10 | <10 | <10 | <10 | <10 |
| T28 | <10 | <10 | <10 | <10 | <10 | <10 |

**Table 5**

| Pseudomonas aeruginosa | | | | | | |
|---|---|---|---|---|---|---|
| | Collagen | | Royal jelly | | Hyaluronic acid | |
| | Comp | Ag | Comp | Ag | Comp | Ag |
| T0 | 945000 | 945000 | 945000 | 945000 | 945000 | 945000 |
| T1 | 98000 | 150 | 300 | 20 | 530 | 20 |
| T7 | 140 | 60 | 30 | 10 | 40 | <10 |
| T14 | 10 | <10 | 20 | <10 | 90 | <10 |
| T28 | <10 | <10 | <10 | <10 | <10 | <10 |

The disclosures in Italian Patent Application No. PD2007A000242 from which this application claims priority are incorporated herein by reference.

## Claims

1. A preservative composition for cosmetic products, comprising:
- 5 to 15% by weight of glycerin with respect to the weight of the cosmetic product,
- 0.01 to 0.5% by weight of chelating compound with respect to the weight of the cosmetic product,
- a dynamic buffer for keeping the pH around 4.50-6.00,
and **characterized in that** it further comprises 0.1 to 1 ppm of electrolytic colloidal silver with respect to the cosmetic product.

2. The composition according to claim 1, wherein the electrolytic colloidal silver is 0.5 ppm with respect to the cosmetic product.

3. The composition according to claim 1, wherein the glycerin is present for 5 to 10% by weight on the weight of the cosmetic product.

4. The composition according to claim 1, wherein the chelating compound is present for 0.05 to 0.3% of the weight of the cosmetic product.

5. The composition according to claim 1 or 4, wherein the chelating compound is ethylenediaminetetraacetic acid and/or salts thereof.

6. The composition according to claim 1, wherein the dynamic buffer keeps the pH around 5.00-5.50.

7. The composition according to claim 1 or 6, wherein the dynamic buffer is constituted by a citric-phosphate buffer.

8. A cosmetic product comprising the preservative composition according to one or more of the preceding claims.

9. The cosmetic product according to claim 8, **characterized in that** it further comprises an additive selected from the group constituted by an acrylic polymer gel and an emulsifier.

10. The cosmetic product according to claim 8, **characterized in that** it comprises an active ingredient selected from the group constituted by collagen, royal jelly and hyaluronic acid.

11. The cosmetic product according to claim 8, **characterized in that** it is in the form of a cream, gel, solution, milk or suspension.

## Patentansprüche

1. Eine Konservierungszusammensetzung für Kosmetikprodukte, die Folgendes umfasst:
- 5 bis 15 Gewichtsprozent Glycerin, bezogen auf das Gewicht des Kosmetikprodukts,
- 0,01 bis 0,5 Gewichtsprozent einer Chelat bildenden Verbindung, bezogen auf das Gewicht des Kosmetikprodukts,
- einen dynamischen Puffer, um den pH bei ungefähr 4,50-6,00 zu halten,
und **dadurch gekennzeichnet, dass** sie weiter 0,1 bis 1 ppm von elektrolytischem Kolloidsilber, bezogen auf das Kosmetikprodukt, umfasst.

2. Die Zusammensetzung gemäß Anspruch 1, worin das elektrolytische Kolloidsilber 0,5 ppm, bezogen auf das Kosmetikprodukt, ist.

3. Die Zusammensetzung gemäß Anspruch 1, worin das Glycerin mit 5 bis 10 Gewichtsprozent im Gewicht des Kosmetikprodukts vorhanden ist.

4. Die Zusammensetzung gemäß Anspruch 1, worin die Chelat bildende Verbindung mit 0,05 bis 0,3 Gewichtsprozent des Kosmetikprodukts vorhanden ist.

5. Die Zusammensetzung gemäß Anspruch 1 oder 4, worin die Chelat bildende Verbindung Ethylendiamintetraessigsäure und/oder-Salze davon ist.

6. Die Zusammensetzung gemäß Anspruch 1, worin der dynamische Puffer den pH bei ungefähr 5,00-5,50 hält.

7. Die Zusammensetzung gemäß Anspruch 1 oder 6, worin der dynamische Puffer aus einem Zitronen-Phosphat-Puffer besteht.

8. Ein Kosmetikprodukt, das die Konservierungszusammensetzung gemäß einem oder mehreren der obigen Ansprüche umfasst.

9. Das Kosmetikprodukt gemäß Anspruch 8, **dadurch gekennzeichnet, dass** es weiter einen Zusatzstoff umfasst, gewählt aus der Gruppe bestehend aus einem Acrylpolymergel und einem Emulgator.

10. Das Kosmetikprodukt gemäß Anspruch 8, **dadurch gekennzeichnet, dass** es einen Wirkstoff umfasst, gewählt aus der Gruppe bestehend aus Kollagen, Gelée royale und Hyaluronsäure.

11. Das Kosmetikprodukt gemäß Anspruch 8, **dadurch gekennzeichnet, dass** es in Form einer Creme, eines Gels, einer Lösung, Milch oder Suspension vorliegt.

## Revendications

1. Composition conservatrice pour produits cosmétiques, comprenant:
- de 5 à 15 % de glycérine, en poids rapporté au poids du produit cosmétique,
- de 0,01 à 0,5 % d'un composé chélatant, en poids rapporté au poids du produit cosmétique,
- et un tampon dynamique, servant à maintenir le pH à une valeur d'à peu près 4,50 à 6,00,
et **caractérisée en ce qu'**elle comprend en outre de l'argent colloïdal électrolytique, en une proportion de 0,1 à 1 ppm par rapport au produit cosmétique.

2. Composition conforme à la revendication 1, dans laquelle l'argent colloïdal électrolytique se trouve en une proportion de 0,5 ppm par rapport au produit cosmétique.

3. Composition conforme à la revendication 1, dans laquelle la glycérine se trouve en une proportion de 5 à 10 %, en poids rapporté au poids du produit cosmétique.

4. Composition conforme à la revendication 1, dans laquelle le composé chélatant se trouve en une proportion de 0,05 à 0,3 %, en poids rapporté au poids du produit cosmétique.

5. Composition conforme à la revendication 1 ou 4, dans laquelle le composé chélatant est de l'acide éthylène-diamine-tétra-acétique et/ou l'un de ses sels.

6. Composition conforme à la revendication 1, dans laquelle le tampon dynamique maintient le pH à une valeur d'à peu près 5,00 à 5,50.

7. Composition conforme à la revendication 1 ou 6, dans laquelle le tampon dynamique est constitué d'un tampon citrate-phosphate.

8. Produit cosmétique comprenant une composition conservatrice conforme à l'une ou plusieurs des revendications précédentes.

9. Produit cosmétique conforme à la revendication 8, **caractérisé en ce qu'**il comprend en outre un adjuvant choisi dans l'ensemble formé par un gel de polymère acrylique et un émulsifiant.

10. Produit cosmétique conforme à la revendication 8, **caractérisé en ce qu'**il comprend un ingrédient actif choisi dans l'ensemble formé par le collagène, la gelée royale et l'acide hyaluronique.

11. Produit cosmétique conforme à la revendication 8, **caractérisé en ce qu'**il se présente sous forme de crème, de gel, de solution, de lait ou de suspension.
